# EUROPEAN PATENT SPECIFICATION

(11) **EP 2 444 081 B1**
(45) Date of publication and mention of the grant of the patent: **08.04.2015**
(21) Application number: 11008419.1
(22) Date of filing: 19.10.2011
(51) Int. Cl.: A61K 31/19, A61K 31/352, A61K 36/185, A61K 36/324, A61P 29/00

(54) **A composition for the treatment of inflammatory diseases comprising boswellic acids and cannabidiol**
Zusammensetzung zur Behandlung von Entzündungskrankheiten enhaltend Cannabidiol und Boswelliasäure
Composition pour le traitement des maladies inflammatoires contenanted de l'acid boswellique et de cannabidiol

(30) Priority: 19.10.2010 CZ 20100764; 30.08.2011 CZ 20110541
(43) Date of publication of application: 25.04.2012
(73) Proprietor: Parenteral, A.S., 170 00 Praha 7 (CZ)
(72) Inventor: Skalicky, Jirí, 50012 Hradec Králové 12 (CZ); Husek, Jirí, 50003 Hradec Králové 3 (CZ); Hofbauerová, Jana, 19000 Praha 9 (CZ); Dittrich, Milan, 50011 Hradec Králové 11 (CZ)
(74) Representative: Fischer, Michael

(56) References cited:
- WO-A1-2006/000867
- US-A1- 2008 255 224
- US-A1- 2009 298 941
- US-A1- 2010 273 895
- MOUSSAIEFF ARIEH ET AL: "Incensole acetate, a novel anti-inflammatory compound isolated from Boswellia resin, inhibits nuclear factor-kappa B activation", MOLECULAR PHARMACOLOGY, AMERICAN SOCIETY FOR PHARMACOLOGY AND EXPERIMENTAL THERAPEUTICS, US, vol. 72, no. 6, 25 September 2007 (2007-09-25), pages 1657-1664, XP009098362, ISSN: 0026-895X
- AMMON H P T: "BOSWELLIASAEUREN (INHALTSSTOFFE DES WEIHRAUCHS) ALS WORKSAME PRINZIPIEN ZUR BEHANDLUNG CHRONISCH ENTZUENDLICHER ERKRANKUNGEN//Boswellic acids (components of frankincense) as the active principle in treatment of chronic inflammatory diseases", WIENER MEDIZINISCHE WOCHENSCHRIFT, SPRINGER WIEN, AT, vol. 152, no. 15-16, 1 January 2002 (2002-01-01), pages 373-378, XP009096034, ISSN: 0043-5341, DOI: 10.1046/J.1563-258X.2002.02056.X
- GUPTA I ET AL: "Effects of Boswellia serrata gum resin in patients with ulcerative colitis.", EUROPEAN JOURNAL OF MEDICAL RESEARCH JAN 1997 LNKD- PUBMED:9049593, vol. 2, no. 1, January 1997 (1997-01), pages 37-43, XP9156006, ISSN: 0949-2321
- GUPTA I ET AL: "Effects of Boswellia serrata gum resin in patients with bronchial asthma: results of a double-blind, placebo-controlled, 6-week clinical study.", EUROPEAN JOURNAL OF MEDICAL RESEARCH 17 NOV 1998 LNKD- PUBMED:9810030, vol. 3, no. 11, 17 November 1998 (1998-11-17), pages 511-514, XP9156005, ISSN: 0949-2321

## Description

### Technical field

1. The present invention relates to a composition for the treatment of inflammatory diseases of the skin, particularly of atopic eczema.

### State of art

Arachidonic acid is released from the cell membrane phospholipids. For example in case of a skin surface injury the skin cell gets injured as well and arachidonic acid is released from its protective membrane which contains phospholipids. Arachidonic acid is then quickly metabolised to eicosanoids - prostaglandins, thromboxanes and leukotrienes that are mostly responsible for allergic, inflammatory, spasmolytic and spasmo-constrictive reactions. As local hormones they affect smooth muscles, participate in elicitation of pain, affect blood coagulation and elicit a number of further mechanisms such as reddening and whitening, tingling and pain, swelling and blood coagulation, asthma, bronchial spasms, etc.

Prostaglandins are oxidized derivates of arachidonic acid that participate in all inflammatory processes in the body. A minimum quantity of these tissue hormones is sufficient to affect particularly smooth muscles, nerve cells, vascular system and reproductive apparatus. Prostaglandins cause vasodillatation that manifests itself with flare, febricity, increased vessel permeability manifested by swelling, they affect pain and fever, reduce arterial pressure of the system and affect aggregation of platelets. They are synthesized in most cells and they are instrumental in stimulation of smooth muscles and metabolism. They affect blood circulation, production of many substances including hormones and digestive juices, blood coagulation, participate in immune and inflammatory processes, increase contractions of uterus musclature, etc. Their effects differ depending on the type of organ in which they are produced.

Leukotrienes originate primarily in leucocytes. They have a very strong broncho-constrictive effect (they cause contraction of smooth muscles). They increase permeability of vessels, have chemoactive and activating effect on leucocytes, especially eosinophiles and monocytes. On the onset of allergic reaction leukotrienes act as very potent broncho-constrictive agents while during the developed allergic reaction they cause proliferation of smooth muscle cells, epithelial cells and fibroplasts. They increase the potency of phagocytosis, inflammatory reactions, hypersensitivness (of asthma) as well as of the immune protection against infections. They influence the emergence or development of particularly inflammatory conditions such as allergic cold, broncho-constrictions, vasodilatation or vasoconstriction, mucociliar clearance, collagen deposition, epithelium cells proliferation, smooth muscles proliferation, mucus formation.

Thromoboxanes are substances produced in platelets. When released they cause vasoconstriction (contraction of vessels) and agglutination of platelets and thus they participate in blood coagulation.

Production of prostaglandines and thromboxanes from arachidonic acid is stimulated by cyclooxygenase while production of leukotrienes from arachidonic acid is stimulated by 5-lipooxygenase.

Inflammatory processes in a human body are treated with steroids (hormones) or with non-hormonal preparations (without presence of hormones).
In case of steroid treatment either corticosteroid hormones (adrenal cortex hormones) or corticoid hormones are applied. The latter are synthetic analogues of corticosteroid hormones synthesised from cholesterol. Corticosteroid or corticoid hormones prevent the release of arachidonic acid from the cell membrane of phospholipids (they inhibit phospholipase) and thus prevent its metabolisation to the tissue forms of eicosanoids. Corticosteroid and corticoid hormones stimulate or inhibit gene transcription directly or by regulating the activity of transcription factors AP-1 (activator protein 1) and NFκB (nuclear factor-κB). The undesirable effects of corticosteroid as well as of corticoid preparations are skin depletion, red cheeks, complicated healing of wounds and minor contusions. The unpleasant side effects are increased appetite leading to weight gain and to uneven fat deposition which is manifested by moon face, abdominal fat or buffalo hump. There have been observed also more serious side effects such as hypertension, bone thinning, hyperglycemia, negative nitrogen balance and infections generally as corticosteroids and corticoids decrease immunity of the organism.

Non-steroid treatment consists of application of chemical preparations with anti-inflammatory effect without the presence of hormones. The best known substance is acetylsalicylic acid and its derivates. It is used as an analgesic for suppressing pain, as an antipyretic for reducing body temperature (fever), as an antirheumatic for its good anti-inflammatory effects and as a preparation soothing skin inflammations. Acetylsalicylic acid blocks the transformation of arachidonic acid to prostaglandins and tromboxanes by permanently inhibiting cyclooxygenase enzyme (COX). Since the acetylsalicylic acid prevents the production of prostaglandins and particularly tromboxanes, it suppresses the symptoms of inflammatory reactions, fever and of other cascade reactions of these two agents. Yet, the acetylsalicylic acid does not cure the cause it only suppresses the natural reaction of organism. The undesirable effect of non-steroid treatment is often the development of stomach ulcers and reduced blood coagulation which might be serious in case of some diseases such as e.g. oesophagus varixes and ulcerous colitis or other latent bleeding conditions.

Inflammatory conditions can be also treated with substances of natural origin.

Such a substance of natural origin with anti-inflammatory effect is Boswellia. It is a tree that grows in India, northern Africa and in the Middle East. Boswellia family plants extract (frankinsence), especially from Boswellia serrata and Boswellia sancta, contains triterpenic pentacyclic compounds called boswellic acids known for anti-inflammatory effects. The extract is mostly applied orally to mitigate arthritic pains and problems. There is also a range of preparations in the form of cream, ointment, paste, microemulsion or gel.

Other such material of natural origin with anti-inflammatory effects is hemp oil obtained especially from the seeds of hemp (Cannabis sativa). It contains a balanced combination of saturated and unsaturated fatty acids. The unsaturated fatty acids are precursors of arachidonic acid that indirectly affect inflammatory processes. The metabolites of unsaturated fatty acids positively affect skin functions, nutrition of skin cells and the skin revitalisation and thus prevent the development of skin diseases. However the anti-inflammatory effect of unsaturated fatty acids and their metabolites is low. The hemp oil contains also a compound called cannabidiol (CBD) which is also known for its anti-inflammatory properties. It emulates the activity of acetylsalicidic acid. Yet, the concentration of CBD in hemp oil is insignificant and the soothing effect of hemp oil is in case of inflammations insufficient.
The objective of the present invention is a composition for the treatment of inflammatory diseases on the basis of natural substances that would have the same or better effect than the anti-inflammatory agents known so far, especially the corticoids and corticosteroids but without the side effects.

### Disclosure of the Invention

A composition for the treatment of inflammatory diseases of the skin, particularly of atopic aczema, removes the current technological deficiencies and at the same time fulfills the objective of the present invention. A composition contains 0,01 - 20 wt % of boswellic acids in the form of extracts or isolates from Boswellia family plants (Burseraceae) or from the salts of these acids and 0,001 - 30 wt % of cannabidiol.

Cannabidiol may be in the form of extract or isolate from Cannabis sativa plant.

The inventors found out that at least the same but rather better anti-inflammatory effect may be obtained with a composition that according to this innovation contains 0,01 - 20 wt % of boswellic acids in the form of extract or isolate from Boswellia family plants (Buseraceae) or from the salts of these acids and 0,0001 - 30 wt % of synthetic cannabidiol, **C₂₁H₃₀O₂** formula I.

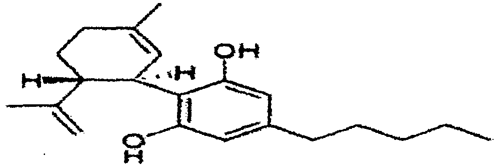

CANNABIDIOL may be of synthetic origin. The production of synthetic cannabidiol is economical as well as technologically less demanding.

The bellow described clinical study shows that synthetic cannabidiol is even more effective that the Cannabis sativa extract containing 12 wt % of cannabidiol.

Cannabidiol melting point is 66 to 67°C and the boiling point is 187 to 190°C at the pressure 266Pa. It is soluble in ethanol, methanol, ether, benzene or chloroform.

The extracts containing boswellic acids or their salts and cannabidiol may be dissolved in lipotropic solvent selected from the group consisting of triglycerides, carbohydrates, alcohols, ketones, esters or ethers.

The extracts containing boswellic acids or their salts and cannabidiol can be dissolved in oil obtained from Cannabis sativa seeds.

The extracts containing boswellic acids or their salts and cannabidiol can be dissolved in hydrophilic solvent selected from the group consisting of aliphatic polar alcohols or their blends with water.

A composition for the treatmentof inflammatory diseases according to the present inventionpresent invention can contain at least one agent selected from the group consisting of co-solvents, emulsifiers and stabilisers to increase the stability of extracts, emulsions, ointments, powders, granulates, pills and pastes as well as accelerators of penetration in order to increase bio-availability of boswellic acids or their salts and of synthetic cannabidiol.

A composition for the treatment of inflammatory diseases according to the present invention can be used for topical administration on skin or mucous membrane, for non-invasive administration in body cavities, for oral administration in human or veterinary medicine to inhibit the symptoms and to treat inflammatory diseases.

A composition for the treatment of inflammatory diseases according to the present invention is based on a composition of boswellic acids obtained in the form of Boswellia family plant extract and cannabidiol obtained in the form of Cannabis sativa plant extract or isolate and/or synthetically produced cannabidiol and of other materials enabling production, increasing stability and bio-availability of anti-inflammatory agents. A composition is prepared in the form of extract, emulsion, ointment, paste, granulate or powder with the use of technologically adjuvant agents suitable for topical administration, administration into body cavities or oral administration (per os).

The amount of components in a composition for the treatment of inflammatory diseases is defined by the range 0,01 - 20 wt % of boswellic acids and by the range 0,0001 - 30 wt % of Cannabidiol. Higher concentrations exceeding the above top limit does not lead to any therapeutic improvement due to the oversaturation of active ingredients Cannabidiol and Boswellic acid on binding receptors and thus the effect on enzymatic reactions of cyclooxygenase, lipooxygenase does not apply. Higher concentrations of effective substances exceeding the above top limits lead to the redundancy of active ingredients in biochemical reactions of the organism. The effect of active ingredients Cannabidiol and Boswellic acid begins to show in low concentrations of the above defined ranges.

A composition for the treatment of inflammatory diseases according to the present invention substantially eliminates or suppresses the undesired effects of corticosteroids/steroids while the effect on the metabolism of arachidonic acid metabolism and the production of its metabolites prostaglandins, tromboxanes and leukotrienes remains the same and their undesired effects are suppressed more effectively.

A composition for the treatment of inflammatory diseases according to the present invention is based on the knowledge of two different processes of anti-inflammatory effect, that of cannabidiol (CBD) and of the Boswellia plant extract containing boswellic acids. Each component has a different anti-inflammatory mechanism. The active ingredients contained in boswellia serrata plant act through the inhibition by 5-lipooxygenase and CBD acts through the inhibition by cyclooxygenase. A composition for the treatment of inflammatory diseases according to the present invention is based on the finding that the effects of the extract from Boswellia plant extract unexpectedly increase and extend in the course of the treatment in combination with concentrated CBD.

CBD inhibits the effect of cyclooxygenase enzyme and prevents the transformation of arachidonic acid to prostagladins and tromboxanes. CBD begins to act already in the concentration of 0,001 wt % and at the same time has fewer undesirable effects than acetylsalycilic acid.

Boswellia family plant extract contains beta-boswellic acid, 3-O-acetyl-boswellic acid, 11-keto-beta-boswellic acid, 3-0-acetyl-11-keto-beta-boswellic and ortho-boswellic acids that act as lipooxygenase inhibitors, especially on enzyme 5-lipooxygenase and thus prevent the production of leukotrienes from arachidinic acid. Boswellic acids act either through direct interaction with lipooxygenase or they block its translocation. They are active particularly in the area of muscles or joints.

Boswellic acids have low biological availability due to the relatively deeper penetration caused by relatively high molecular weight. They are not capable of deeper penetration through skin or mucous membrane. The molecular weight of boswellic acids reaches the approximate value of 500. In order to achieve sufficient biological availability of boswellic acids in the organism it is thus advisable to use hem oil as a good natural transdermal enhancer which has high penetration and bonding properties.

By blending boswellic acids (3-O-acetyl-beta-boswellic and 3-O-acetyl-keto-beta-boswellic) and CBD, possibly under the temperature of 30 - 45°C, we obtain a novel composition. This process leads to the development of a novel transport form of active ingredients in one complex and the availability of active ingredients (boswellic acids and SBD) in the organism is achieved which ensures fast synchronous start of their activity. This synergic effect is close to that of corticoids and corticosteroids yet without the side-effects. They prevent in the whole range the biochemical reaction of the transformation of arachidonic acid to undesirable eikosanoids the same as corticoids. Combination of these two anti-inflammatory mechanisms results in a composition with an increased synergic effect preventing the development of leukotrienes from the released arachidonic acid by the inhibition of 5-lipooxygenase (boswellic acid from boswellia serrata)as well as preventing the inhibition of cyclooxygenase in the metabolic transformation of arachidonic acid to prostaglandins and tromboxanes (cannabidiol). The resulting effect is more powerful and extensive in suppressing inflammatory and allergic mechanisms than the effect of each of these preparations - boswellic acids extract and cannabidiol -individually as they complement one another.

The combined and synergic effect of the two materials for example results in the elimination of reddening skin after the application on atopic eczema as the effects of leukotrienes causing vessel dilatation and promoting blood circulation in the skin leading to reddening are blocked by boswellic acids. If only CBD is applied, the reddening occurs as cannabidiol cannot block leukotriens.

The active ingredients of a composition for the treatment of inflammatory diseases according to the present invention broaden the anti-inflammatory spectrum and bring new properties which cannot be found in either of the active ingredients and the effect of which is unexpected. The synergic effect of the compositions consists in the complex action of the two components on inflammatory processes. The described anti-inflammatory effect of a composition can be strengthened by suitable effective enhancers. The optimum enhancer is hemp oil. Hemp oil has an increased ability to transport other active ingredients into deeper layers of skin, mucous membrane or other barriers as well as the ability to bind a wider spectrum of biobioactive ingredients such as cytokines, CD mnonoclonal antibodies, arachidonic acid derivates, low molecular weight peptides, antibiotics, antimicrobic substances, chemotherapeuticals, peptides, nucleotides and other substances.

This oil itself or blended with other substances is a highly effective enhancer of penetration or absorption. Hemp oil significantly increases availability of boswellic acids and cannabidiol in the deeper layers of skin down to subcutis. The speedy absorption in the skin is facilitated by hemp oil linoleic and linolenic acids. The structure of intercellular matter is quickly and effectively changed, its viscosity and polarity reduced which creates favourable parametres for a faster process of passive diffusion of boswellic acids and cannabidiol through intercellular space not only to the deep skin layers but also to subcutis structures. This mechanism produces desired local effect in the tissue close to the preparation administration and thus extends and enhances anti-inflammatory effect in the treatment of skin inflammations (eczema, atopic eczema and other types of dermatitis including allergic symptoms).

A composition for the treatment according to the present invention is an effective non-steroid preparation in the treatment of inflammatory diseases and of other commonly known inflammations of the skin, including painfuly sprained muscles, muscle contractions, swellings, etc.

Cannabis sativa extract (Cannabidiol extractum)can be isolated in a concentrated form by extracting dried green parts of Cannabis sativa with liquid carbon dioxide or other solvents (ether, ethanol, petrolether and other solvents used for extraction). In this way we obtain Cannabis sativa extractum (Cannabidiol extractum) as a material to be used directly or as a raw material to produce pure CBD in crystalic form.

Cannabidiol in the crystalic form (isolate) can be prepared by dissolving the extracted mass of Cannabis sativa extractum (Cannabidiol extract)in the form of slurry (1 kilogram) in ethanol (4 kilograms) under reflux temperature. The undissolved materials should be filtered out and the solvent distilled. The residue should be distilled with water steam (appr. 8 litres of distilled liquor) to eliminate terpene impurities (will remain in the distilation tank). The solid materials in the distilled liquor are filtered out and subsequently dissolved in petrolether (boiling point 30-60 °C)and this solution is extracted three times with water. By distilling the solvent we obtain material "fred oil" which is then further purified. This material is dissolved in ethanol to which active coal is added (appr. 1 gramme per 1000 millilitres of ethanol), the solution is refluxed for 5 minutes and filtered while still hot. This procedure is repeated three times. By distillation of ethanol we obtain cannabidiol as white to colourless crystals with the melting point 66 - 67°C.

### Examples

### Example 1

Aerosol anti-inflammatory formulation with hemp oil enriched with CBD and Boswellia serrata extract applicable on skin. The components are listed in weight percentages. The components are mixed in the order mentioned.
Use: eczema, dermatitis, psoriasis

| Component | Conc. (%) |
|---|---|
| Isopropylmyristate | 51,00 |
| Cannabis sativa oil | 25,00 |
| Cyclometicone | 9,50 |
| Polyisobutene hydrogenated | 9,49 |
| Boswellia serrata | 4,80 |
| Alpha bisabolol | 0,20 |
| Cannabis sativa extract | 0,01 |

### Example 2

Emulgel anti-inflammatory preparation with hemp oil enriched with hemp extract with CBD and Boswellia serrata extract applicable on skin and mucous membrane. The components are listed in weight percentages.

| Component | Conc. (%) |
|---|---|
| Aqua | 49,00 |
| Cannabis sativa oil | 18,00 |
| Alcohol SD | 9,00 |
| Cannabis sativa extract | 8,00 |
| Glycerin | 5,80 |
| Boswellia serrata extract water soluble | 4,30 |
| Polysorbate 60 | 2,00 |
| Benzylalcohol | 0,90 |
| Carbomer | 0,60 |
| Disodium EDTA | 0,20 |
| Sodium hydroxide sol. 10% | 2,10 |
| Dehydroacetic acid | 0,10 |

Procedure: Emulsion w/o is prepared by homogenizing oil in water with polysorbate, adding ethanol, benzylalcohol, dehydroacetic acid and the extracts are then mixed. Carbomer is dispersed in the solution and after it has swelled up solution of sodium hydroxide is added.
Use: dermatitis, the formulation soothes mucous membrane inflammations, heals varicous ulcers, joint swelling, it has anti-inflammatory effect on arthritis.

### Example 3

Preparation in the form of cream with hemp oil enriched by cannabidiol (CBD) in hemp extract and by Boswellia serrata extract applicable on skin and mucous membrane. The components are listed in weight percentages:

| Component | Conc. |
|---|---|
| Aqua | 52,10 |
| Cetyl Alcohol | 8,20 |
| Glycerin | 8,00 |
| Paraffinum perliquidum | 7,80 |
| Petrolatum | 5,00 |
| Stearyl alcohol | 3,00 |
| Boswellia serrata extract | 3,00 |
| Lanolin | 2,50 |
| Bees wax | 2,50 |
| Propylene glycol | 1,70 |
| PEG-60 castor oil hydrogenated | 1,50 |
| Monoglyceride laurate | 1,30 |
| Caprylyl glycol | 0,95 |
| Carbomer | 0,25 |
| Disodium EDTA | 0,15 |
| Sodium hydroxide sol. 10% | 1,25 |
| Cannabis sativa extract | 0,55 |
| Dehydroacetic acid | 0,25 |

Procedure: Water, fatty alcohols, vaseline, wax, sheep wool fat and hydrogenated ethoxylated castor oil were mixed and heated to 75°C. Stirred and cooled to 40°C. Solutions of extracts were added in liquid paraffin and homogenized. The remaining components except for the hydroxide were mixed and added to the mixure. Finally hydroxide was added and mixed. Use: serious inflammation of muscular attachments, swellings, arthritis, eczema, dermatitis, psoriasis, burns; the preparation relieves pain.

### Example 4

Oinment with anti-inflammatory effect with the Boswellia serrata extract and Cannabis sativa haulm extract applicable on skin. The components are listed in weight percentages.

| Component | Conc. % |
|---|---|
| Petrolatum | 38,00 |
| Ethyl oleate | 20,00 |
| Boswellia serrata extract | 15,00 |
| Isopropyl stearate | 15,00 |
| Lanolin hydrogenated | 6,00 |
| Cetyl and stearyl alcohol | 4,45 |
| Benzyl alcohol | 0,80 |
| Cannabis sativa extract | 0,55 |
| Dehydroacetic acid | 0,20 |

Procedure: Vaseline with hydrogenated lanoline and fatty alcohols were heated until the melting point, isopropylmyristate was added, mixed and finally solution of extract in ethyloleate with benzylalcohol and dehydroacetic acid was added.
Use: strong and long lasting anti-inflammatory deep reaching effect, e.g. for the treatment of arthritis, muscle inflammations, eczema and psoriasis. The preparation soothes dermatitis, seborrea, acne.

### Example 5

Paste with Boswellia serrata with hemp haulm (cyme) extract for oral administration. The components are listed in weight percentages.

| Component | Conc. (%) |
|---|---|
| Amygdalus dulcis oil | 72,00 |
| Boswellia serrata extract | 22,00 |
| Silica anhydrous | 5,00 |
| Tocoferol acetate | 0,94 |
| Butylhydroxyanisole | 0,05 |
| Cannabis sativa extractum | 0,01 |

Procedure: Amygdalus dulcis oil was homogenized with pyrogenous silicic oxide. The gel gradually mixed with extracts, tocoferol acetate and butylhydroxyanisole. The paste can be filled in capsules.
Use: Crohn disease, ulcerative colitis, bronchial asthma, gastric ulcer.

### Example 6

Powder containing water soluble Boswellia serrata extract, cannabidiol (CBD) isolate and pycnogenol to be packed in capsules for peroral, rectal or vaginal administration or to be used for the production of suppositories. The components are listed in weight percentages.

| Component | Conc. (%) |
|---|---|
| Mannitol | 80,00 |
| Boswellia serrata extract water soluble | 10,00 |
| Cannabidiol | 8,00 |
| Pycnogenol | 1,00 |
| Silica | 1,00 |

Procedure: All components with pyrogenous silicic oxide were homogenized. The powder can be filled in capsules or dispersed in lipotropic suppository base.
Use: Crohn disease, ulcerative colitis, bronchial asthma, gastric ulcer, vaginal inflammations.

### Example 7 (Hydrophilic lotion)

| Component | Concentration (%weight) |
|---|---|
| Phase A | |
| Aqua | 62,45 |
| PEG-100 behenate | 2,70 |
| Stearic acid | 7,30 |
| Sorbitan stearate | 2,60 |
| Glycerin | 3,40 |
| Benzylalcohol | 0,95 |
| Dehydroacetic acid | 0,15 |
| Phenoxyethanol | 0,45 |
| | |

| Phase B | |
|---|---|
| Cannabidiol | 1,33 |
| Cannabis sativa seed oil | 7,00 |
| Boswellia serrata extract | 0,65 |
| Squalene | 4,00 |
| Vaselinum album | 4,00 |
| Isopropyl myristate | 3,00 |
| Butylhydroxyanisole | 0,02 |

### Procedure:

Phase A was heated to 75°C and cooled while stirring intensively.

Phase B was prepared separately by heating up all the components to 40°C while stirring until they have dissolved. Phase B was added to Phase A cooled down to 40°C and stirred until dissolution.
The emulsion was homogenized in colloidal mill.

### Example 8 (Occlusive iontment)

| Component | Concentration (% weight.) |
|---|---|
| Phase A | |
| Cera alba | 9,50 |
| Vaselinum album | 38,00 |
| Lanolinum | 12,50 |
| Butylhydroxytoluene | 0,05 |
| Cannabidiol | 2,80 |
| Cannabis sativa seed oil | 10,00 |
| Boswellia serrata extract | 1,95 |
| Squalene | 8,00 |
| Isopropyl myristate | 5,40 |
| Sorbitan stearate | 3,00 |
| | |

| Phase B | |
|---|---|
| Glycerin | 3,50 |
| Propylenglykol | 5,00 |
| Methylparaben | 0,10 |
| Propylpylparaben | 0,05 |
| Phenoxyethanol | 0,65 |

### Procedure:

Phase A was heated to 65°C and cooled while stirring intensively.

Phase B was prepared separately at room temperature and stirred until the components dissolved.
Phase B was added to Phase A at the temperature 50°C and mixed intensively till the temperature dropped to 25°C.

### Example 9 (Peroral capsules)

| Components | Concentration(% weight) |
|---|---|
| Cannabidiol | 10,50 |
| Boswellia serrata extract | 22,00 |
| Ascorbyl palmitane | 2,00 |
| Cannabis sativa seed oil | 60,00 |
| Silica colloidalis anhydrica | 5,50 |

### Procedure:

Mix intensively all the components until they have formed colloidal glutinous mixture.

The gel is filled in soft gelatinous capsules of the volume 0,25 ml 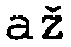 1,23 ml.

### Clinical studies

In 2009 and 2010 extensive studies were conducted that confirm better and faster effectiveness of the novel composition according to the present invention in comparison with the treatment of each of the material separately.

The following studies of the effect of a composition on atopic eczema were conducted:
1) Study of the natural substance BOSWELLIA SERRATA,
2) Study of the Cannabis sativa green mass extract containing CBD
3) Study of synthetic CANNABIDIOL
4) Study of Cannabis sativa green mass extract containing CBD and BOSWELLIA SERRATA
5) Study of a composition based upon synthetic CANNABIDIOL and BOSWELLIA SERRATA

### 1) Study of the natural substance BOSWELLIA SERRATA

STUDY OF THE EFFECT OF NATURAL SUBSTANCE BOSWELLIA SERRATA ON ATOPIC ECZEMA: COMPOSITION OF THE OINTMENT - 94 WT % WHITE VASELINE, 6 WT % BOSWELLIA SERRATA

**ATOPIC ECZEMA**

| Patient No. | Sex | Start date of administration | Examination Week 1 | Examination Week 2 | Examination Week 3 | Examination Week 4 | Examination Week 5 |
|---|---|---|---|---|---|---|---|
| | | | | | | | |
| Pat.No. 1 | Male | 6.4.2009 | - | * | + | + | ++ |
| Pat.No. 2 | Male | 6.4.2009 | 0 | + | ++ | * | +++ |
| Pat.No.3 | Female | 8.4.2009 | + | ++ | * | +++ | +++ |
| Pat.No.4 | Male | 8.4.2009 | + | ++ | * | +++ | +++ |
| Pat.No.5 | Female | 10.4.2009 | - | + | ++ | * | +++ |
| Pat.No.6 | Female | 10.4.2009 | + | * | ++ | * | ++ |
| Pat.No.7 | Female | 14.4.2009 | 0 | * | + | ++ | ++ |
| Pat.No. 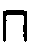 | Female | 14.4.2009 | + | ++ | * | - | ++ |
| Pat.No.9 | Male | 14.4.2009 | 0 | + | * | ++ | +++ |
| Pat.No.10 | ŹEN 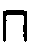 | 20.4.2009 | 0 | + | ++ | ++ | +++ |
| Pat.No.11 | Male | 20.4.2009 | 0 | ++ | * | +++ | +++ |
| Pat.No.12 | Female | 17.4.2009 | - | + | + | ++ | +++ |
| | | | | | | | |
| | | | 3-;5-0;4- +; | 1-;1-0; 6- +;4- ++; | 4-+;8- 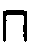 ++; | 1--;1- +; 7- ++;3- +++; | 4-++,8-+++, |

| | | | | | | | |
|---|---|---|---|---|---|---|---|
| LEGEND: IMPAIRED - NOT IMPROVED 0 CONDITION same as one week ago * SLIGHTLY IMPROVED + SIGNIFICANTLY IMPROVED ++ NOT ACTIVE +++ | | | | | | | |

All patients were older than 18 years and they agreed with the research. One month prior to the study their condition was not treated.
The selected patients suffered from acute atopic eczema symptoms. Boswellia Serrata - 65 % extract dissolved in heated vaseline.

### ASSESSMENT:

First week - symptoms of atopic eczema the same to slightly improved, in three cases the symptoms got worse
Second week - symptoms of atopic eczema mostly slightly improved (6) to significantly improved (4)
Third week - symptoms of atopic eczema significantly improved in most cases (8)
Fourth week - symptoms of atopic eczema most significantly improved (7), first symptoms of not a ctive eczema (3)
Fifth week - symptoms of atopic eczema mostly not active (8)
We did not assess the reasons for impaired condition (4) as they were Not the subject of our study.

### CONCLUSIONS:

**From the third week the symptoms of atopic eczema were significantly improving**

### 2) Study of the extract from Cannabis sativa green mass containing CBD

**STUDY OF THE EFFECT OF CANNABIS SATIVA GREEN MASS EXTRACT (THC <0,3%) CONTAINING CBD ON ATOPIC ECZEMA IN THE FORM OF OINTMENT - 99,75 WHILE VASELINE AND 0,25% OF HEMP EXTRACT CONTAINING 12% CBD (300mg/kg)**

**ATOPIC ECZEMA**

| **Patient No.** | **Sex** | **Start date of administration** | **Examination Week 1** | **Examination Week 2** | **Examination Week 3** | **Examination Week 4** | **Examination Week 5** |
|---|---|---|---|---|---|---|---|
| | | | | | | | |
| Pat.No. 1 | Female | 20.5.2009 | 0 | + | ++ | +++ | +++ |
| Pat.No. 2 | Female | 20.5.2009 | 0 | + | ++ | +++ | +++ |
| Pat.No.3 | Female | 20.5.2009 | 0 | * | + | ++ | ++ |
| Pat.No.4 | Female | 122.5.2009 | 0 | + | ++ | +++ | +++ |
| Pat.No.5 | Male | 22.5.2009 | + | * | ++ | ++ | +++ |
| Pat.No.6 | Female | 22.5.2009 | 0 | * | + | ++ | +++ |
| Pat.No.7 | Male | 27.5.2009 | + | * | ++ | ++ | +++ |
| Pat.No.8 | Male | 27.5.2009 | + | * | + | ++ | +++ |
| Pat.No.9 | Female | 27.5.2009 | + | ++ | +++ | +++ | +++ |
| Pat.No.10 | Female | 29.5.2009 | 0 | + | ++ | +++ | - |
| Pat.No.11 | Male | 29.5.2009 | + | ++ | +++ | +++ | +++ |
| Pat.No.12 | Male | 29.5.2009 | - | + | ++ | - | ++ |
| Pat.No.13 | Male | 2.6.2009 | 0 | + | ++ | ++ | +++ |
| Pat.No.14 | Female | 2.6.2009 | 0 | + | ++ | +++ | +++ |
| Pat.No.15 | Female | 2.6.2009 | + | ++ | +++ | +++ | +++ |
| | | | | | | | |
| | | | 2--;8- 0; 6- +; | 2-0;10- +; 3- ++; | 3-+;9- ++; 3- +++; | 1--;6- ++; 8- +++; | 1--;2- ++; 12- +++; |

| | | | | | | | |
|---|---|---|---|---|---|---|---|
| **LEGEND:** IMPAIRED - NOT IMPROVED 0 CONDITION same as one week ago * SLIGHTLY IMPROVED + SIGNIFICANTLY IMPROVED ++ NOT ACTIVE +++ | | | | | | | |

All patients were older than 18 years and they agreed with the research.
One month prior to the study their condition was not treated. The selected patients suffered from acute atopic eczema symptoms.
Cannabidiol - extract from technical hemp green mass with 12% of CBD.

### ASSESSMENT:

First week - only minor improvement (6)
Second week - significant progress towards SLIGHT IMPROVEMENT (10)
Third week - visible effect SIGNIFICANT IMPROVEMENT (9) with signs of NO VISIBLE ACTIVITY (3)
Fourth week - predominantly NO VISIBLE ACTIVITY (8) and SIGNIFICANT IMPROVEMENT (6)
Fifth week - predominantly NO VISIBLE ACTIVITY (12).

### CONCLUSIONS:

From the second week the skin condition was visibly improving, from the third week the atopic symptoms were disappearing with most of the patients. Maximum improvement was registered in the fourth and fifth week.

### 3) Study of the synthetic CANNABIDIOL

THE STUDY OF EFFECT OF SYNTHETIC CANNABIDIOL ON ATOPIC ECZEMA IN THE FORM OF OINTMENT - 99,97% WHILE VASELINE, 0,03% CBD (300mg/kg).

**ATOPIC ECZEMA**

| Patient No. | Sex | Start date of administration | Examination Week 1 | Examination Week 2 | Examination Week 3 | Examination Week 4 | Examination Week 5 |
|---|---|---|---|---|---|---|---|
| Pat.No. 1 | Male | 4.4.2011 | + | ++ | ++ | ++ | ++ |
| Pat.No. 2 | Male | 4.4.2011 | 0 | + | ++ | +++ | +++ |
| Pat.No.3 | Female | 4.4.2011 | + | ++ | ++ | ++ | +++ |
| Pat.No.4 | Female | 4.4.2011 | 0 | + | ++ | +++ | ++ |
| Pat.No.5 | Male | 7.4.2011 | + | * | +++ | ++ | +++ |
| Pat.No.6 | Female | 7.4.2011 | 0 | + | + | +++ | +++ |
| Pat.No.7 | Female | 7.4.2011 | + | * | ++ | +++ | +++ |
| Pat.No.8 | Male | 19.4.2011 | + | ++ | ++ | ++ | +++ |
| Pat.No.9 | Female | 19.4.2011 | + | ++ | ++ | +++ | +++ |
| Pat.No.10 | Female | 19.4.2011 | 0 | + | +++ | +++ | +++ |
| Pat.No.11 | Male | 21.4.2011 | + | ++ | ++ | +++ | +++ |
| Pat.No.12 | Male | 21.4.2011 | + | + | ++ | +++ | +++ |
| Pat.No.13 | Female | 21.4.2011 | 0 | + | ++ | +++ | +++ |
| Pat.No.14 | Female | 121.4.2011 | 0 | + | +++ | ++ | +++ |
| Pat.No.15 | Female | 21.4.2011 | + | ++ | +++ | +++ | +++ |
| | | | | | | | |
| | | | 6- 0;9- +; | 9- +;6- ++; | 1-+;10- ++; 4- +++; | 5- ++; 10- +++; | 2- ++; 13- +++; |

| | | | | | | | |
|---|---|---|---|---|---|---|---|
| **LEGEND:** IMPAIRED - NOT IMPROVED 0 CONDITION same as one week ago * SLIGHTLY IMPROVED + SIGNIFICANTLY IMPROVED ++ NOT ACTIVE +++ | | | | | | | |

### CBD = CANNABIDIOL

All patients were older than 18 years and they agreed with the research.
One month prior to the study their condition was not treated. The selected patients suffered from acute atopic eczema symptoms.
Cannabidiol - synthetic CBD - 90% pure

### ASSESSMENT:

First week - no impairment compared to the extract, higher rate of minor improvement (9)
Second week - compared to the extract more significant development towards SLIGHT IMPROVEMENT (10) plus significant improvement (6)
Third week - visible effect - SIGNIFICANT IMPROVEMENT (10) with signs of NOT ACTIVE (4)
Fourth week - NOT ACTIVE (5), prevailing condition SIGNIFICANT IMPROVEMENT (10)
Fifth week - prevailing condition NOT ACTIVE (13) No case of deteoration was registered.

### CONCLUSIONS:

The study has proved that the synthetically produced cannabidiol is more effective than cannabidiol contained in the extract from Cannabis sativa green mass. The results indicate that synthetically produced cannabiodiol is more effective already from the first week of application. No deteoration of the condition was registered with our patients.

### 4) Study of the compound of extract from the technical hemp green mass containing CBD and BOSWELLIA SERRATA

STUDY OF THE COMPOUND OF BOCANOL ON ATOPIC ECZEMA IN THE FORM OF OINTMENT COMPOSED OF 93,75% WHITE VASELINE, 0,25% EXTRACT FROM THE CANNABIS SATIVA GREEN MASS (300mg/kg CBD) AND 6% BOSWELLIA SERRATA

**ATOPIC ECZEMA**

| Patient No. | Sex | Start date of administration | Examination Week 1 | Examination Week 2 | Examination Week 3 | Examination Week 4 | Examination Week 5 |
|---|---|---|---|---|---|---|---|
| | | | | | | | |
| Pat.No. 1 | Female | 10.8.2009 | 0 | + | +++ | 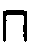 | * |
| Pat.No. 2 | Female | 10.8.2009 | + | ++ | +++ | * | * |
| Pat.No.3 | Female | 12.8.2009 | + | ++ | +++ | * | * |
| Pat.No.4 | Male | 12.8.2009 | + | ++ | +++ | * | * |
| Pat.No.5 | Female | 12.8.2009 | + | + | - | - | ++ |
| Pat.No.6 | Male | 17.8.2009 | - | + | ++ | +++ | * |
| Pat.No.7 | Female | 1 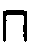 .8.2009 | 0 | ++ | ++ | +++ | * |
| Pat.No.8 | Female | 17.8.2009 | + | ++ | ++ | * | * |
| Pat.No.9 | Male | 18.8.2009 | + | +++ | * | 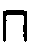 | * |
| Pat.No.10 | Male | 18.8.2009 | + | ++ | +++ | * | ++ |
| Pat.No.11 | Female | 20.8.2009 | + | ++ | +++ | * | * |
| Pat.No.12 | Female | 20.8.2009 | + | ++ | +++ | * | * |
| | | | | | | | |
| | | | 1--;2-0;9-+; | 3-+;8-++;1-+++; | 1--;2-++;9-+++; | 1--;11-+++; | 2-+ 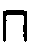 ;10-+++; |

| | | | | | | | |
|---|---|---|---|---|---|---|---|
| LEGEND: IMPAIRED - NOT IMPROVED 0 CONDITION same as one week ago * SLIGHTLY IMPROVED + SIGNIFICANTLY IMPROVED ++ NOT ACTIVE +++ | | | | | | | |

**BOCANOL** - COMPOUND/MIXTURE OF GREEN EXTRACT FROM TECHNICAL HEMP (THC < 0,3%) IN CONCENTRATION 0,25% AND FROM BOSWELLIA SERRATA IN CONCENTRATION 6%.

All patients were older than 18 years and they agreed with the research.
One month prior to the study their condition was not treated.
The selected patients suffered from acute atopic eczema symptoms.

### ASSESSMENT:

FIRST Week - COMPARED TO PREVIOUS STUDIES ALMOST ALL PATIENTS SLIGHTLY IMPROVED (9).
SECOND Week - rapid onset of Bocanol effect leading to SIGNIFICANT IMPROVEMENT (8) and to NO ACTIVITY (1).
THIRD Week - significant symptoms of eczema withdrawal resulting in NO ACTIVITY (9)
FOURTH AND FIFTH Week - NO ACTIVITY condition prevails (11-10)

### CONCLUSIONS:

The composition/mixture of the extract from Cannabis sativa green mass an effectiveness and a faster start of healing process compared to the individual components in the relevant mixtures (extract from Cannabis sativa green mass and from Boswellia Serrata acids).

### 5) Study of a composition from synthetic CANNABIDIOL and BOSWELLIA SERRATA

STUDY ON BOCANOL EFFECT ON ATOPIC ECZEMA IN THE FORM OF OINTMENT COMPOSED OF 93,97% WHITTE VASELINE, 0,03% SYNTHETIC CANNABIDIOL (300 mg/kg) and 6% BOSWELLIA SERRATA.

**A TOPICKÝ EKZÉM**

| Patient No. | Sex | Start date of administration | Examination Week 1 | Examination Week 2 | Examination Week 3 | Examination Week 4 | Examination Week 5 |
|---|---|---|---|---|---|---|---|
| | | | | | | | |
| Pat.No. 1 | Male | 3.5.2011 | + | + | ++ | +++ | * |
| Pat.No. 2 | Male | 3.5.2011 | + | +++ | * | * | * |
| Pat.No.3 | Female | 3.5.2011 | + | ++ | +++ | * | * |
| Pat.No.4 | Female | 10.5.2011 | + | ++ | +++ | * | * |
| Pat.No.5 | Female | 10.5.2011 | + | ++ | +++ | +++ | * |
| Pat.No.6 | Male | 12.5.2011 | + | ++ | +++ | +++ | * |
| Pat.No.7 | Male | 12.5.2011 | 0 | + | +++ | +++ | * |
| Pat.No.8 | Female | 12.5.2011 | + | ++ | +++ | * | * |
| Pat.No.9 | Female | 16.5.2011 | + | +++ | * | * | * |
| Pat.No.10 | Female | 16.5.2011 | + | ++ | +++ | * | ++ |
| Pat.No.11 | Female | 16.5.2011 | + | +++ | +++ | * | * |
| Pat.No.12 | Female | 16.5.2011 | + | +++ | +++ | * | * |
| | | | | | | | |
| | | | 1-0;11-+; | 2-+;6-++;4-+++; | 1-++;11-+++; | 12-+++; | 12-+++; |

| | | | | | | | |
|---|---|---|---|---|---|---|---|
| **LEGEND:** IMPAIRED - NOT IMPROVED 0 CONDITION same as one week ago * SLIGHTLY IMPROVED + SIGNIFICANTLY IMPROVED ++ NOT ACTIVE +++ | | | | | | | |

BOCANOL - COMPOUND/MIXTURE OF SYNTHETIC CANNABIDIOL (concentration 0,03%) AND BOSWELLIA SERRATA (concentration 6%).

All patients were older than 18 years and they agreed with the research.
One month prior to the study their condition was not treated.
The selected patients suffered from acute atopic eczema symptoms.

### ASSESSMENT:

FIRST Week - rapid effect leading to SLIGHT IMPROVEMENT (11)
SECOND Week - rapid Bocanol effect continues leading to SIGNIFICANT IMPROVEMENT (6) and NO ACTIVITY (4)
THIRD Week - significant symptoms of eczema withdrawal leading to NO ACTIVITY condition (11)
FOURTH and FIFTH Week - prevailing significant symptoms of NO ACTIVITY condition (12)

### CONCLUSIONS:

In this study the composition/mixture (BOCANOL) of synthetic cannabidiol and Boswellia Serrata unambiguously proved better effectiveness and earlier start of healing process than BOCANOL containing the hemp extract. It can thus be presumed that synthetically produced cannabidiol, unlike that produced from the hemp green mass extract, does not contain any other potentially irritating substances.

The composition with synthetic cannabidiol which is the subject of the invention received the best rating in clinical tests.

The results of clinical tests univocally prove high synergic effect of the novel composition manifested by earlier and statistically clearly significant treatment effect. The composition demonstrated soothing effect already after one week of application unlike the individual substances the effect of which became manisfest only after three weeks.

In order to ensure objective assessment the concentrations of specific active ingredients were the same as in the studies with only one active ingredient. The blending was intentionally simple with only white pharmaceutical vaseline to prevent distortion of the assessment due to the potential effect of other ingredients.

### Clinical use

Synergic effect of a composition based upon cannabidiol (CBD) and boswellic acids relieves the inflammatory diseases of the skin. The ratio of cannabidiol and boswellic acids depends on the type of inflammation to be cured by the formulation according to this invention. If we wish to suppress leukotrienes more than other agents, the content of boswellic acids that is of Boswellia serrata extract, should be bigger. If we want to suppress more the undesirable effect of prostaglandines and tromboxanes, the content of cannabidiol should be increased. A composition for the treatment of inflammatory diseases of the skin can be successfully applied to treat the following diseases: eczema, atopic eczema, psoriasis, acne, allergic dermatitis, contact dermatitis, seborrhea.

A composition for the treatment of inflammatory diseases according to the present invention can be used externally (skin, mucous membrane) and it can be produced in the form of oil, cream, ointment, pills and capsules.

## Claims

1. A composition for the treatment of inflammatory diseases of the skin, particularly of atopic eczema,
**characterised by** that
it contains 0,01 - 20 wt % of boswellic acids weight in the form of extract or isolate from Boswellia family plants (Burseraceae)or from the salts of these acids and 0,001 - 30 wt % of cannabidiol.

2. A composition for the treatment of inflammatory diseases of the skin according to the claim 1
**characterised by** that
Cannabidiol is in the form of extract or isolate from Cannabis sativa family plant.

3. A composition for the treatment of inflammatory diseases of the skin according to the claim 1,
**characterised by** that
cannabidiol is of synthetic origin.

4. A composition for the treatment of inflammatory diseases of the skin according to any of the claims 1 to 3
**characterised by** that
the extracts containing boswellic acids or their salts and cannabidiol are dissolved in lipotropic solvent selected from a group consisting of triglycerides, hydrocarbons, alcohols, ketones, esters or ethers.

5. A composition for the treatment of inflammatory diseases of the skin according to any of the claims 1 to 4
**characterised by** that
the extracts containing boswellic acids or their salts and cannabidiol are dissolved in the oil from Cannabis sativa seeds.

6. A composition for the treatment of inflammatory diseases of the skin according to any of the claims 1 to 5
**characterised by** that
the extracts containing boswellic acids or their salts and cannabidiol are dissolved in hydrophilic solvent selected from the group consisting of aliphatic polar alcohols or their mixtures with water.

7. A composition for the treatment of inflammatory diseases of the skin according to any of the claims 1 - 6
**characterised by** that
it contains at least one of the agent selected from the group consisting of co-solvents, emulsifiers and stabilisers for the increased stability of solutions, emulsions, ointments, powders, granulates, pills and pastes and by enhancers of penetrations for the increased bioavailability of boswellic acids or their salts and of synthetic cannabidiol.

8. The use of a composition for the treatment ofinflammatory diseases of the skin, particularly of atopic eczema according to claims 1 to 7
for topical administration on skin to suppress the symptoms and to treat inflamatory effect of the disease.

## Patentansprüche

1. Komposition für die Behandlung von entzündlichen Erkrankungen der Haut, insbesondere von atopischen Ekzemen,
**dadurch gekennzeichnet**, dass
sie 0,01 - 20 Vol% Boswelliasäuren in Form eines Extraktes oder Isolats, das aus den Pflanzen der Gattung Boswellia Familie Burseraceae oder Salzen dieser Säuren und 0,001 - 30 Vol% Cannabidiol stammt.

2. Komposition für die Behandlung von entzündlichen Erkrankungen der Haut gemäß dem Anspruch 1,
**dadurch gekennzeichnet,** dass
das Cannabidiol in der Form des Extraktes oder Isolats aus der Pflanze der Gattung Cannabis sativa ist.

3. Komposition für die Behandlung von entzündlichen Erkrankungen der Haut gemäß dem Anspruch 1,
**dadurch gekennzeichnet,** dass
das Cannabidiol synthetischen Ursprungs ist.

4. Komposition für die Behandlung von entzündlichen Erkrankungen der Haut gemäß einem der Ansprüche 1 bis 3,
**dadurch gekennzeichnet**, dass
die Extrakte, die Boswelliasäuren oder ihre Salze und das Cannabidiol enthalten, in einem lipophilen Lösungsmittel aufgelöst sind, das aus der Gruppe ausgewählt ist, die durch Triglyceride, Kohlenwasserstoffe, Alkohole, Ketone, Ester oder Äther gebildet wird.

5. Komposition für die Behandlung von entzündlichen Erkrankungen der Haut gemäß einem der Ansprüche 1 bis 4,
**dadurch gekennzeichnet,** dass
die Extrakte, die Boswelliasäuren oder ihre Salze und das Cannabidiol enthalten, im Öl aus den Samen eines Saat-Hanfs Cannabis sativa aufgelöst sind.

6. Komposition für die Behandlung von entzündlichen Erkrankungen der Haut gemäß einem der Ansprüche 1 bis 5,
**dadurch gekennzeichnet**, dass
die Extrakte, die Boswelliasäuren oder ihre Salze und das Cannabidiol enthalten, im hydrophilen Lösungsmittel aufgelöst sind, das aus der Gruppe ausgewählt ist, die durch aliphatische polare Alkohole oder ihre Wassergemische gebildet wird.

7. Komposition für die Behandlung von entzündlichen Erkrankungen der Haut gemäß einem der Ansprüche 1 - 6,
**dadurch gekennzeichnet**, dass
sie mindestens einen Stoff aus der Gruppe enthält, die durch Kosolvente, Emulgatoren und Stabilisatoren für die Erhöhung der Stabilität von Lösungen, Emulsionen, Salben, Pulver, Granulaten, Tabletten und Pasten, und durch Penetrationsbeschleuniger für die Erhöhung der biologischen Verfügbarkeit der Boswelliasäuren oder ihrer Salze und des synthetischen Cannabidiols gebildet wird.

8. Verwendung der Komposition für die Behandlung von entzündlichen Erkrankungen der Haut, insbesondere von atopischen Ekzemen gemäß den Ansprüchen 1 bis 7, für topische Applikation auf die Haut für die Minderung der Symptome und Behandlung von entzündlichen Erkrankungen.

## Revendications

1. Composition destinée au traitement des maladies infectieuses de la peau, et plus particulièrement des eczémas atopiques,
**caractérisée en ce que que**
elle contient 0,01 - 20 % de masse d'acides boswelliques sous forme d'extrait ou d'isolat provenant de plantes de l'espèce du Boswellia, famille des Burséracées, ou de sels de ces acides et 0,001 - 30 % de masse de cannabidiol.

2. Composition destinée au traitement des maladies infectieuses de la peau selon la revendication 1,
**caractérisée en ce que que**
le cannabidiol est sous forme d'extrait ou d'isolat de plantes de l'espèce du chanvre cultivé (Cannabis sativa).

3. Composition destinée au traitement des maladies infectieuses de la peau selon la revendication 1,
**caractérisée en ce que que**
le cannabidiol est d'origine synthétique.

4. Composition destinée au traitement des maladies infectieuses de la peau selon une des revendications 1 à 3,
**caractérisée en ce que que**
les extraits contenant des acides boswelliques ou leurs sels et du cannabidiol sont dissouts dans un solvant lipophile sélectionné au sein d'un groupe formé de triglycérides, d'hydrates de carbone, d'alcools, de cétones, d'esters ou d'éthers.

5. Composition destinée au traitement des maladies infectieuses de la peau selon une des revendications 1 à 4,
**caractérisée en ce que que**
les extraits contenant des acides boswelliques ou leurs sels et du cannabidiol sont dissouts dans de l'huile de graines de cannabis technique semé Cannabis sativa.

6. Composition destinée au traitement des maladies infectieuses de la peau selon une des revendications 1 à 5,
**caractérisée en ce que que**
les extraits contenant des acides boswelliques ou leurs sels et du cannabidiol sont dissouts dans un solvant hydrophile sélectionné au sein d'un groupe formé d'alcools aliphatiques polaires ou de leurs mélanges avec de l'eau.

7. Composition destinée au traitement des maladies infectieuses de la peau selon une des revendications 1 à 6,
**caractérisée en ce que que**
elle contient au moins une des substances appartenant à un groupe formé de co-solvants, d'émulsifiants et de stabilisants destinés à la préparation d'améliorateurs de stabilité des solutions, des émulsions, des pommades, des poudres, des granulats, des comprimés et des pâtes, et d'accélérateurs de pénétration visant à améliorer la biodisponibilité des acides boswelliques, de leurs sels et du cannabidiol synthétique.

8. Emploi d'une composition destinée au traitement des maladies infectieuses de la peau, et plus particulièrement des eczémas atopiques selon des revendications 1 à 7, pour une application topique sur la peau en vue d'atténuer les manifestations des maladies infectieuses et de traiter ces maladies.
